# EUROPÄISCHE PATENTSCHRIFT

(11) **EP 1 216 724 B1**
(45) Veröffentlichungstag und Bekanntmachung des Hinweises auf die Patenterteilung: **22.12.2004**
(21) Anmeldenummer: 01250442.9
(22) Anmeldetag: 17.12.2001
(51) Int. Cl.: A61N 1/39

(54) **Vorrichtung zur Behandlung einer Fibrillation mindestens einer Kammer eines Herzens**
Apparatus for the treatment of fibrillation of at least one heart chamber
Appareil de traitement de la fibrillation d'au moins une chambre cardiaque

(30) Priorität: 18.12.2000 DE 10064597
(43) Veröffentlichungstag der Anmeldung: 26.06.2002
(73) Patentinhaber: BIOTRONIK Mess- und Therapiegeräte GmbH & Co Ingenieurbüro Berlin, 12359 Berlin (DE)
(72) Erfinder: Thong, Tran, Portland, Oregon 97229 (US); Shekhar, Mrigank, Vancouver, WA 98662 (US); Nigam, Indra B, Tigard, Oregon 97223 (US); Schaldach, Max, . (DE); Flach, Erhard, 12305 Berlin (DE)
(74) Vertreter: Eisenführ, Speiser & Partner

(56) Entgegenhaltungen:
- EP-A- 0 838 234
- US-A- 6 068 651

## Beschreibung

Die vorliegende Erfindung betrifft eine Vorrichtung zur Behandlung einer Fibrillation mindestens einer Kammer eines Herzens mit einem Fibrillationsdetektor zum Erfassen einer Fibrillation, einem Defibrillator zum Defibrillieren der Kammer des Herzens, wobei der Defibrillator mit dem Fibrillationsdetektor verbunden ist und dazu ausgebildet ist, im Anschluss an ein Zeitintervall nach der Erfassung der Fibrillation eine Defibrillation durchzuführen, einer Warneinrichtung, welche mit dem Fibrillationsdetektor verbunden ist und dazu ausgebildet ist, ein Warnsignal abzugeben, wenn eine Fibrillation erfasst wurde, und einem Steuermittel mit einem von einem Patienten ansteuerbaren Steuereingang, wobei das Steuermittel mit dem Defibrillator verbunden ist und dazu ausgebildet ist, den Zeitpunkt einer Defibrillation zu verzögern, wenn das Steuermittel ein entsprechendes Signal über den Steuereingang empfängt.

Die Fibrillation gehört zu den Herzrhythmusstörungen und bezeichnet eine unregelmäßige und unkoordinierte Kontraktion der Muskelfasern des Herzens. Man unterscheidet zwischen atrialer und ventrikulärer Fibrillation je nachdem, ob die Fibrillation im Atrium und Ventrikel eines Herzens auftritt. Atriale Fibrillationen gehören zu den am häufigsten auftretenden Herzrhythmusstörungen. Sie sind jedoch nicht unmittelbar lebensbedrohlich, so dass eine Behandlung nicht sofort durchgeführt werden muss. Das US-Patent 6,068,651 der Pacesetter Inc. schlägt deshalb eine implantierbare Vorrichtung zur Behandlung von Fibrillationen vor, die ein Patientensteuermittel aufweist, das es dem Patienten erlaubt, den Zeitpunkt der Defibrillationsbehandlung zu steuern. Der Patient wird zunächst mittels einer Warneinrichtung davon in Kenntnis gesetzt, dass eine atriale Fibrillation vorliegt. Der Patient hat dann die Möglichkeit, während eines vorbestimmten Zeitraums eine Defibrillation des Atrium selbst auszulösen oder den Zeitpunkt einer Defibrillation weiter hinauszuzögern. Ein überraschender und möglicherweise schmerzhafter Eintritt einer Fibrillationsbehandlung kann somit für den Patienten vermieden werden.

Es kann jedoch mit Risiken behaftet sein, den Zeitpunkt für die Behandlung einer Fibrillation von einem Patienten bestimmen zu lassen. Denn eine atriale Fibrillation kann eine ventrikuläre Fibrillation mit der Zeit nach sich ziehen oder selbst eine solche Steigerung erfahren, dass eine akute Gefährdung des Patienten besteht. Ferner kann sie von einer ventrikulären Tachykardie begleitet sein was ebenfalls nicht ungefährlich für den Patienten ist.

Es ist daher Aufgabe der vorliegenden Erfindung, eine Vorrichtung zur Behandlung einer Fibrillation mindestens einer Kammer eines Herzens der eingangs genannten Art bereitzustellen, die die vorstehenden Nachteile behebt.

Die Vorrichtung zur Behandlung einer Fibrillation gemäß dem beigefügten Anspruch 1 löst diese Aufgabe. Die Vorrichtung umfasst einen Fibrillationsdetektor zum Erfassen einer Fibrillation, einen Defibrillator zum Defibrillieren der Kammer des Herzens, wobei der Defibrillator mit dem Fibrillationsdetektor verbunden ist und dazu ausgebildet ist, im Anschluss an ein Zeitintervall nach der Erfassung der Fibrillation eine Defibrillation durchzuführen, eine Warneinrichtung, welche mit dem Fibrillationsdetektor verbunden ist und dazu ausgebildet ist, ein Warnsignal abzugeben, wenn eine Fibrillation erfasst wurde und ein Steuermittel mit einem von einem Patienten ansteuerbaren Steuereingang, wobei das Steuermittel mit dem Defibrillator verbunden und dazu ausgebildet ist, den Zeitpunkt einer Defibrillation zu verzögern, wenn das Steuermittel ein entsprechendes Signal über den Steuereingang empfängt. Ferner ist die erfindungsgemäße Vorrichtung dadurch gekennzeichnet, dass sie einen Zustandsdetektor umfasst, der dazu ausgebildet ist, einen hämodynamischen Zustand des Herzens zu erfassen, und das Steuermittel mit dem Zustandsdetektor verbunden und dazu ausgebildet ist, ein Verzögern des Zeitpunkts der Defibrillation zu unterbinden, wenn der Zustandsdetektor einen vorgegebenen hämodynamischen Zustand erfasst. Nach wie vor ist der Patient in der Lage, mittels des Steuermittels den Zeitpunkt einer Defibrillation hinauszuzögern. Dies geschieht jedoch nur, wenn der Zustandsdetektor keinen vorgegebenen hämodynamischen Zustand erfasst. Unter den vorgegebenen hämodynamischen Zuständen sind solche Zustände gemeint, die eine baldige oder sofortige Behandlung des Herzens erfordern. Wenn der Zustandsdetektor einen solchen hämodynamischen Zustand erfasst, so lässt das Steuermittel eine Verzögerung der Defibrillation nicht zu. Die Defibrillation wird also durchgeführt, selbst wenn der Patient sie zu verzögern sucht. Grundsätzlich ist es dem Patienten also möglich, den Zeitpunkt der Defibrillation hinauszuzögern. Wenn jedoch eine akute Gefährdung des Patienten vorliegt, findet die Defibrillation unverzögert statt. Damit wird dem Patienten weiterhin erlaubt, den Zeitpunkt der Defibrillation zu bestimmen, ohne jedoch eine Gefährdung des Patienten in Kauf nehmen zu müssen.

Vorzugsweise ist der Fibrillationsdetektor dazu ausgebildet, eine atriale Fibrillation zu erfassen, und der Defibrillator ist dazu ausgebildet, eine atriale Fibrillation zu behandeln. Atriale Fibrillationen sind in der Regel nicht kritisch, so dass es sinnvoll ist, einem Patienten die Möglichkeit zu geben, den Zeitpunkt der Behandlung einer derartigen Fibrillation zu verschieben. Voraussetzung dafür ist, dass die erfindungsgemäße Vorrichtung in die Lage versetzt wird, eine atriale Fibrillation zu erfassen und zu behandeln. Wenn jedoch die atriale Fibrillation den vorgegebenen hämodynamischen Zustand herbeiführt, so verhindert das Steuermittel die Verzögerung der Fibrillationsbehandlung.

Bevorzugt ist der Fibrillationsdetetktor dazu ausgebildet, eine ventrikuläre Fibrillation zu erfassen, und der Defibrillator ist vorzugsweise auch dazu ausgebildet, eine ventrikuläre Fibrillation zu behandeln. Es ist so möglich, sowohl ventrikuläre als auch atriale Fibrillationen zu erkennen und zu behandeln.

Die Warneinrichtung ist bevorzugt mit dem Zustandsdetektor verbunden und dazu ausgebildet, ein erstes Warnsignal auszugeben, wenn der vorgegebene hämodynamische Zustand und eine Fibrillation erfasst wurden, und ein zweites Warnsignal auszugeben, wenn kein vorgegebener hämodynamischer Zustand und eine Fibrillation erfasst wurden. Die Defibrillation des Herzens erfolgt ohne Verzögerung, wenn der vorgegebene hämodynamische Zustand und eine Fibrillation erfasst werden. Das erste Warnsignal gibt dem Patienten demnach zu erkennen, dass eine Defibrillation unmittelbar bevorsteht. Der Patient weiß zudem, dass eine Verzögerung der Behandlung ausgeschlossen ist. Das zweite Warnsignal deutet darauf hin, dass eine Fibrillation vorliegt, die verzögert werden kann. Denn die Verzögerung der Fibrillation wird durch das Steuermittel nicht unterbunden, wenn kein vorgegebener hämodynamischen Zustand vorliegt. Der Patient erhält somit durch die beiden unterschiedlichen Warnsignale Kenntnis davon, in welchem Zustand er sich befindet. Er kann sich und seine Umgebung damit besser auf die Behandlung vorbereiten.

Der Defibrillator ist vorzugsweise dazu ausgebildet, vor der Defibrillation ein Schmerzmittel und/oder Beruhigungsmittel abzugeben. Zusätzlich oder alternativ kann die Vorrichtung zur Behandlung einer Fibrillation auch eine Schmerztherapieeinheit aufweisen, die mit dem Steuermittel sowie mit Nervenelektroden verbunden und dazu ausgebildet ist, über die Nervenelektroden elektrische Impulse abzugeben, die geeignet sind, Schmerzempfindungen zu betäuben. Die Defibrillation des Herzens ist in der Regel für den Patienten sehr schmerzhaft und zudem stellt sie eine außergewöhnliche und beunruhigende Situation für den Patienten dar, in der er zeitweise nicht unerheblichen Schmerzen ausgesetzt wird. Die Verabreichung von Schmerz- und Beruhigungsmitteln kann verhindern, dass der Patient in einen kritischen, stressbedingten Schockzustand gerät.

Der Zustandsdetektor ist vorzugsweise dazu ausgebildet, den vorgegebenen hämodynamischen Zustand anhand eines oder mehrerer Indikatoren zu ermitteln. Dazu ist der Zustandsdetektor vorzugsweise mit dem Fibrillationsdetektor verbunden und ausgebildet, die ventrikuläre Fibrillation als einen der Indikatoren oder den Indikator zu erfassen. Ventrikuläre Fibrillationen sind lebensgefährlich und führen nach kurzer Dauer zum Tod. Sie zeigen demnach einen Zustand an, der eine sofortige Behandlung erfordert. Erkennt der Zustandsdetektor das Vorliegen einer ventrikulären Fibrillation, so kann er eine Verzögerung der Defibrillation des Ventrikels verhindern. Ferner ist der Zustandsdetektor vorzugsweise dazu ausgebildet, als den Indikator oder als einen der Indikatoren eine Herzleistung zu erfassen. Die Herzleistung gibt das Volumen an Blut an, die das Herz pro Zeiteinheit durch den Körper pumpt. Ein starker Abfall der Herzleistung führt zu einer Unterversorgung des Körpers mit Blut, was schließlich zum Tode führen kann. Deshalb ist die Herzleistung ein wichtiger Indikator für den hämodynamischen Zustand des Patienten. Fällt die Herzleistung unter einen bestimmten, vorgegebenen Schwellenwert, so kann das Steuermittel eine Verzögerung der Defibrillation unterbinden. Vorzugsweise wird die Herzleistung mittels epikardialer oder intrakardialer Impedanzmessungen von dem Zustandsdetektor erfasst. Zur Impedanzmessung werden zumindest zwei Elektroden eingesetzt, durch die ein Wechselstrom durch das Gewebe geleitet wird. Während der Wechselstrom durch das Gewebe fließt, entsteht eine Potentialdifferenz zwischen den Elektroden, die proportional zur Impedanz der Elektroden ist. Der Strom fließt vorzugsweise durch Körpermaterial mit einer hohen Leitfähigkeit wie Blut. Weniger Strom fließt durch Muskeln, die eine mittlere Leitfähigkeit aufweisen, während Fett, Luft und Knochen eine sehr niedrige Leitfähigkeit aufweisen. Ferner ist die Impedanz eine Funktion der Leitfähigkeit und der Querschnittsfläche des leitenden Körpers. Durch Impedanzmessungen zwischen Elektroden, die epikardial oder endokardial am Herzen befestigt sind, kann somit das Volumen des Blutes gemessen werden, das sich in der Herzkammer befindet. Die zyklischen Änderungen der Impedanz des Herzens sind somit auf die Füllung und Entleerung der Herzkammern zurückzuführen. Damit besteht ein Maß für die Herzleistung. Schließlich ist der Zustandsdetektor vorzugsweise dazu ausgebildet, als den Indikator oder als einen der Indikatoren einen Blutdruck zu erfassen. Der Blutdruck ist bekanntermaßen ein Maß für den hämodynamischen Zustand des Patienten.

Vorzugsweise weist die Vorrichtung zur Behandlung einer Fibrillation-insbesondere das Steuermittel - Mittel zum manuellen Auslösen einer atrialen Defibrillation durch den Patienten auch für den Fall auf, dass der Fibrillationsdetektor noch keine Fibrillation detektiert hat. Dies ermöglicht es dem Patienten von sich aus eine atriale Defibrillation auszulösen, wenn er sich unwohl fühlt und z.B. Schwindelgefühle hat, die auf eine atriale Fibrillation hinweisen, auf die der Fibrillationsdetektor noch nicht angesprochen hat.

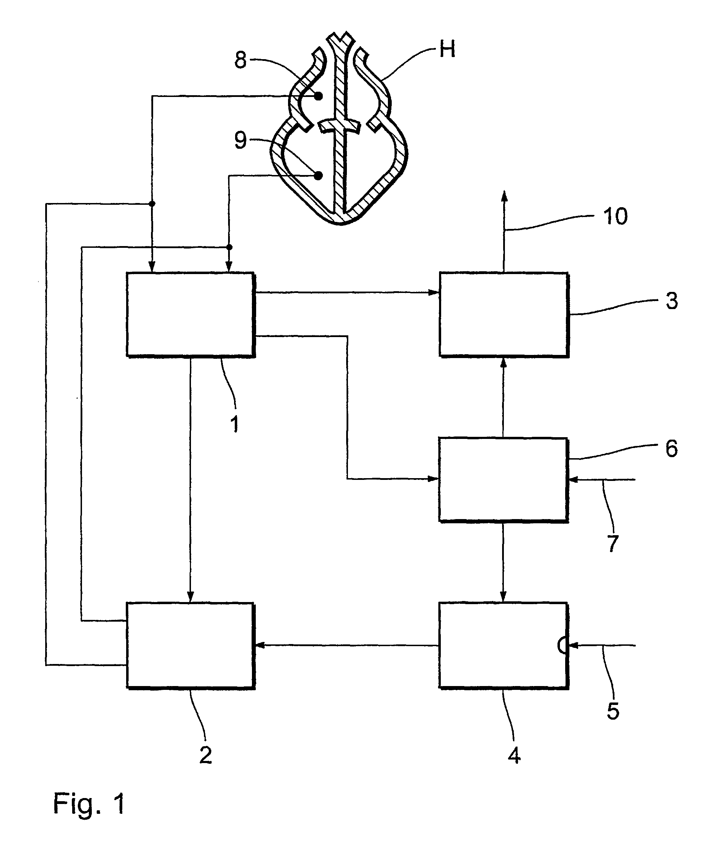

Ein bevorzugtes Ausführungsbeispiel der vorliegenden Erfindung soll nachfolgend anhand der beigefügten Figur beschrieben werden. Es zeigt
- Figur 1: eine Vorrichtung zur Behandlung einer Fibrillation mindestens einer Kammer eines Herzens gemäß dem ersten Ausführungsbeispiel der vorliegenden Erfindung.

Bezugszeichen 1 kennzeichnet einen Fibrillationsdetektor in Figur 1. Dieser besitzt zwei Eingänge zum Anschließen von je einer Elektrode, die im Ventrikel und im Atrium des zu überwachenden Herzens plaziert werden. Die aus dem Ventrikel bzw. Atrium des Herzens stammenden elektrischen Signale kennzeichnen die Aktivität des Atriums bzw. Ventrikels. Der Fibrillationsdetektor 1 ist dazu ausgebildet, anhand der über die jeweiligen Eingänge empfangenen elektrischen Signale eine Fibrillation des Atriums oder Ventrikels zu erfassen.

Der Fibrillationsdetektor 1 ist ausgangsseitig mit einem Defibrillator 2 sowie mit Elektroden 8 und 9 verbunden. Über die Elektroden 8 und 9 erhält der Fibrillationsdetektor 1 elektrische Signale von einem Herzen H, die nach Art eines Elektrokardiogramms eine Fibrillation des Atriums oder des Ventrikels kennzeichnen können. Der Fibrillationsdetektor 1 ist außerdem mit einer Warneinrichtung 3 verbunden und sendet ein Signal an die Warneinrichtung 3, wenn er atriale oder ventrikuläre Fibrillationen erfasst.

Ferner ist der Fibrillationsdetektor 1 mit einem Zustandsdetektor 6 verbunden, der ein Signal von dem Fibrillationsdetektor 1 empfängt, wenn dieser eine ventrikuläre Fibrillation erfasst. Ein solches Signal veranlasst den Zustandsdetektor 6 wiederum dazu, ein Signal an die Warneinrichtung 3 zu übertragen, mit der er ebenfalls verbunden ist.

Empfängt die Warneinrichtung 3 im Wesentlichen zeitgleich ein Signal von dem Zustandsdetektor 6 und dem Fibrillationsdetektor 1, so gibt sie ein erstes Wamsignal an den Patienten ab. Als Warnsignal können elektrische oder akustische Signale dienen, das Warnsignal muss zumindest von dem Patienten wahrgenommen werden können. Das erste Warnsignal signalisiert sowohl das Vorhandensein einer Fibrillation als auch eines kritischen Zustandes. Eine ventrikuläre Fibrillation ist in aller Regel kritisch für einen Patienten, so dass die Warneinrichtung 3 bei Vorliegen einer ventrikulären Fibrillation das erste Warnsignal an den Patienten abgibt. Wenn jedoch die Warneinrichtung 3 lediglich ein Signal von dem Fibrillationsdetektor 1 empfängt, so gibt sie ein zweites Warnsignal ab. Dieses signalisiert für den Patienten ein Vorhandensein einer Fibrillation aber keines kritischen Zustandes.

Der Zustandsdetektor 6 kann ferner weitere Eingänge aufweisen, über die er Signale 7 empfängt, die einen kritischen hämodynamischen Zustand des Patienten anzeigen. Der Zustandsdetektor 6 sendet sowohl ein Signal an die Warneinrichtung 3 als auch an ein Steuermittel 4, mit dem er verbunden ist, wenn ein vorgegebener hämodynamischer Zustand erfasst wird.

Das Steuermittel 4 besitzt einen Steuereingang 5 zum Empfangen von Steuersignalen. Die Steuersignale können elektrischer, elektromagnetischer oder akustischer Natur sein. Das Steuermittel 4 ist ferner mit dem Defibrillator 2 verbunden. Empfängt das Steuermittel 4 ein Signal von dem Zustandsdetektor 6, so sendet es kein Signal an den Defibrillator 2, unabhängig davon, ob ein Signal zeitgleich über den Steuereingang empfangen wurde. Der Defibrillator 2 ist wiederum mit den Elektroden 8 und 9 verbunden, die im Atrium und Ventrikel des Herzens H liegen. Ferner ist der Defibrillator 2 mit dem Fibrillationsdetektor 1 verbunden. Der Fibrillationsdetektor 1 signalisiert dem Defibrillator 2, ob eine atriale und/oder ventrikuläre Fibrillation vorliegt. Wenn der Defibrillator 2 ein solches Signal von dem Fibrillationsdetektor 1 und kein Signal von dem Steuermittel 4 empfängt, so sendet er elektrische Impulse 8 und 9 über die Elektroden an das Atrium oder Ventrikel, um diese zu defibrillieren. Bei Vorliegen einer atrialen Fibrillation und keiner ventrikulären Fibrillation wird lediglich das Atrium von dem Defibrillator 2 defibrilliert. Dementsprechend verfährt der Defibrillator 2 bei Vorliegen einer ventrikulären Fibrillation. Empfängt der Defibrillator 2 jedoch ein Signal von dem Steuermittel 4, so verzögert er die Abgabe von elektrischen Impulsen zur Defibrillation um einen gewissen Zeitraum. Dieser Zeitraum ist in dem von dem Steuermittel empfangenen Signal kodiert und wird von dem Defibrillator 2 gelesen.

## Patentansprüche

1. Vorrichtung zur Behandlung einer Fibrillation mindestens einer Kammer eines Herzens mit einem Fibrillationsdetektor (1) zum Erfassen einer Fibrillation, einem Defibrillator (2) zum Defibrillieren der Kammer des Herzens, wobei der Defibrillator mit dem Fibrillationsdetektor (1) verbunden ist und dazu ausgebildet ist, im Anschluss an ein Zeitintervall nach der Erfassung der Fibrillation eine Defibrillation durchzuführen, einer Warneinrichtung (3), welche mit dem Fibrillationsdetektor (1) verbunden ist und dazu ausgebildet ist, ein Warnsignal abzugeben, wenn eine Fibrillation erfasst wurde, und einem Steuermittel (4) mit einem von einem Patienten ansteuerbaren Steuereingang (5), wobei das Steuermittel mit dem Defibrillator (2) verbunden und dazu ausgebildet ist, den Zeitpunkt einer Defibrillation zu verzögern, wenn das Steuermittel (4) ein entsprechendes Signal über den Steuereingang (5) empfängt, **dadurch gekennzeichnet, dass** die Vorrichtung einen Zustandsdetektor (6) umfasst, der dazu ausgebildet ist, einen hämodynamischen Zustand des Herzens zu erfassen, und das Steuermittel (4) mit dem Zustandsdetektor (6) verbunden und dazu ausgebildet ist, ein Verzögern des Zeitpunkts der Defibrillation zu unterbinden, wenn der Zustandsdetektor (6) einen vorgegebenen hämodynamischen Zustand erfasst.

2. Vorrichtung nach Anspruch 1, **dadurch gekennzeichnet, dass** der Fibrillationsdetektor (1) dazu ausgebildet ist, eine atriale Fibrillation zu erfassen und der Defibrillator (2) dazu ausgebildet ist, eine atriale Fibrillation zu behandeln.

3. Vorrichtung nach Anspruch 1 oder 2, **dadurch gekennzeichnet, dass** der Fibrillationsdetektor (1) dazu ausgebildet ist, eine ventrikuläre Fibrillation zu erfassen.

4. Vorrichtung nach Anspruch 3, **dadurch gekennzeichnet, dass** der Defibrillator (2) dazu ausgebildet ist, eine ventrikuläre Fibrillation zu behandeln.

5. Vorrichtung nach einem der vorstehenden Ansprüche, **dadurch gekennzeichnet, dass** die Warneinrichtung (3) mit dem Zustandsdetektor (6) verbunden ist und dazu ausgebildet ist, ein erstes Warnsignal auszugeben, wenn der vorgegebene hämodynamische Zustand und eine Fibrillation erfasst wurden, und ein zweites Warnsignal auszugeben, wenn kein vorgegebener hämodynamischer Zustand und eine Fibrillation erfasst wurden.

6. Vorrichtung nach einem der vorstehenden Ansprüche, **dadurch gekennzeichnet, dass** der Defibrillator (2) dazu ausgebildet ist, vor der Defibrillation ein Schmerzmittel und/oder Beruhigungsmittel abzugeben.

7. Vorrichtung nach einem der vorstehenden Ansprüche, **gekennzeichnet durch** eine Schmerztherapieeinheit, die mit dem Steuermittel (4) sowie mit Nervenelektroden verbunden und dazu ausgebildet ist, über die Nervenelektroden elektrische Impulse abzugeben, die geeignet sind, Schmerzempfindungen zu betäuben.

8. Vorrichtung nach einem der vorstehenden Ansprüche, **dadurch gekennzeichnet, dass** der Zustandsdetektor (6) dazu ausgebildet ist, den vorgegebenen hämodynamischen Zustand anhand eines oder mehrerer Indikatoren zu ermitteln.

9. Vorrichtung nach Ansprüchen 3 und 8, **dadurch gekennzeichnet, dass** der Zustandsdetektor (6) mit dem Fibrillationsdetektor (1) verbunden und dazu ausgebildet ist, als den Indikator oder als einen der Indikatoren die ventrikuläre Fibrillation zu erfassen.

10. Vorrichtung nach Anspruch 8 oder 9, **dadurch gekennzeichnet, dass** der Zustandsdetektor (6) dazu ausgebildet ist, als den Indikator oder als einen der Indikatoren eine Herzleistung zu erfassen.

11. Vorrichtung nach Anspruch 10, **dadurch gekennzeichnet, dass** der Zustandsdetektor (6) dazu ausgebildet ist, die Herzleistung mittels epikardialer oder endokardialer Impedanzmessungen zu erfassen.

12. Vorrichtung nach Anspruch 8, 9, 10 oder 11, **dadurch gekennzeichnet, dass** der Zustandsdetektor (6) dazu ausgebildet ist, als den Indikator oder als einen der Indikatoren einen Blutdruck zu erfassen.

13. Vorrichtung nach einem der vorstehenden Ansprüche, **gekennzeichnet durch** Mittel zum manuellen Auslösen einer atrialen Defibrillation von außerhalb des Körpers, die zumindest mittelbar mit dem Defibrillator (2) verbunden und ausgebildet sind, eine Defibrillation von einem Patienten auch in dem Fall auslösen zu lassen, in dem der Fibrillationsdetektor (1) noch keine Fibrillation detektiert hat.

14. Vorrichtung nach Anspruch 13, **dadurch gekennzeichnet, dass** das Steuermittel (4) die Mittel zum manuellen Auslösen einer atrialen Defibrillation umfasst

## Claims

1. Apparatus for treating fibrillation of at least one chamber of a heart comprising a fibrillation detector (1) for detecting a fibrillation, a defibrillator (2) for defibrillating the chamber of the heart, wherein the defibrillator is connected to the fibrillation detector (1) and is adapted to effect defibrillation subsequently to a time interval after detection of the fibrillation, a warning device (3) which is connected to the fibrillation detector (1) and which is adapted to deliver a warning signal when a fibrillation has been detected, and a control means (4) having a control input (5) actuable by a patient, wherein the control means is connected to the defibrillator (2) and is adapted to delay the time of a defibrillation when the control means (4) receives a corresponding signal by way of the control input (5), **characterised in that** the apparatus includes a condition detector (6) which is adapted to detect a haemodynamic condition of the heart, and the control means (4) is connected to the condition detector (6) and is adapted to prevent a delay in the time of defibrillation when the condition detector (6) detects a predetermined haemodynamic condition.

2. Apparatus according to claim 1, **characterised in that** the fibrillation detector (1) is adapted to detect atrial fibrillation and the defibrillator (2) is adapted to treat atrial fibrillation.

3. Apparatus according to either claim 1 or claim 2, **characterised in that** the fibrillation detector (1) is adapted to detect ventricular fibrillation.

4. Apparatus according to claim 3, **characterised in that** the defibrillator (2) is adapted to treat ventricular fibrillation.

5. Apparatus according to any one of the preceding claims, **characterised in that** the warning device (3) is connected to the condition detector (6) and is adapted to output a first warning signal when the predetermined haemodynamic condition and a fibrillation have been detected, and to output a second warning signal when no predetermined haemodynamic condition and a fibrillation have been detected.

6. Apparatus according to any one of the preceding claims, **characterised in that** the defibrillator (2) is adapted to deliver a pain killer and/or a tranquilliser prior to defibrillation.

7. Apparatus according to any one of the preceding claims, **characterised by** a pain therapy unit which is connected to the control means (4) and to nerve electrodes and is adapted to deliver by way of the nerve electrodes electrical pulses which are capable of numbing pain sensations.

8. Apparatus according to any one of the preceding claims, **characterised in that** the condition detector (6) is adapted to ascertain the predetermined haemodynamic condition on the basis of one or more indicators.

9. Apparatus according to claim 3 and claim 8, **characterised in that** the condition detector (6) is connected to the fibrillation detector (1) and is adapted to detect ventricular fibrillation as the indicator or as one of the indicators.

10. Apparatus according to either claim 8 or claim 9, **characterised in that** the condition detector (6) is adapted to detect heart output as the indicator or as one of the indicators.

11. Apparatus according to claim 10, **characterised in that** the condition detector (6) is adapted to detect heart output by means of epicardial or endocardial impedance measurements.

12. Apparatus according to claim 8, claim 9, claim 10 or claim 11, **characterised in that** the condition detector (6) is adapted to detect a blood pressure as the indicator or as one of the indicators.

13. Apparatus according to any one of the preceding claims, **characterised by** means for manually initiating atrial defibrillation from outside the body, which are at least indirectly connected to the defibrillator (2) and are adapted to cause initiation of defibrillation by a patient even in the situation in which the fibrillation detector (1) has not yet detected fibrillation.

14. Apparatus according to claim 13, **characterised in that** the control means (4) includes the means for manual initiation of atrial defibrillation.

## Revendications

1. Dispositif pour le traitement d'une fibrillation d'au moins une cavité d'un coeur avec un détecteur de fibrillation (1) pour détecter une fibrillation, un défibrillateur (2) pour défibriller la cavité du coeur, où le défibrillateur est relié au détecteur de fibrillation (1) et est agencé pour réaliser une fibrillation à la suite d'un intervalle de temps après la détection de la fibrillation, un dispositif d'avertissement (3) qui est relié au détecteur de fibrillation (1) et qui est agencé pour délivrer un signal d'avertissement quand une fibrillation a été détectée, et un moyen de commande (4) avec une entrée de commande (5) qui peut être commandée par un patient, où le moyen de commande est relié au défibrillateur (2) et est agencé pour retarder le moment d'une défibrillation quand le moyen de commande (4) reçoit un signal correspondant par le biais de l'entrée de signal (5), **caractérisé en ce que** le dispositif comprend un détecteur d'état (6) qui est agencé pour détecter un état hémodynamique du coeur, et le moyen de commande (4) est relié au détecteur d'état (6) et est agencé pour empêcher un retard du moment de la défibrillation quand le détecteur d'état (6) détecte un état hémodynamique prédéterminé.

2. Dispositif selon la revendication 1, **caractérisé en ce que** le détecteur de fibrillation (1) est agencé pour détecter une fibrillation auriculaire et le défibrillateur (2) est agencé pour traiter une fibrillation auriculaire.

3. Dispositif selon la revendication 1 ou 2, **caractérisé en ce que** le détecteur de fibrillation (1) est agencé pour détecter une fibrillation ventriculaire.

4. Dispositif selon la revendication 3, **caractérisé en ce que** le défibrillateur (2) est agencé pour traiter une fibrillation ventriculaire.

5. Dispositif selon l'une des revendications précédentes, **caractérisé en ce que** le dispositif d'avertissement (3) est relié au détecteur d'état (6) et est agencé pour délivrer un premier signal d'avertissement quand l'état hémodynamique prédéterminé et une fibrillation ont été détectés, et pour délivrer un deuxième signal d'avertissement quand aucun état hémodynamique prédéterminé n'a été détecté et quand une fibrillation a été détectée.

6. Dispositif selon l'une des revendications précédentes, **caractérisé en ce que** le défibrillateur (2) est agencé pour délivrer un analgésique et/ou un tranquillisant avant la défibrillation.

7. Dispositif selon l'une des revendications précédentes, **caractérisé par** une unité de thérapie de la douleur qui est reliée au moyen de commande (4) et à des neuroélectrodes et qui est agencée pour délivrer, par le biais des neuroélectrodes, des impulsions électriques qui conviennent pour atténuer les sensations douloureuses.

8. Dispositif selon l'une des revendications précédentes, **caractérisé en ce que** le détecteur d'état (6) est agencé pour déterminer l'état hémodynamique prédéterminé au moyen d'un ou plusieurs indicateurs.

9. Dispositif selon les revendications 3 et 8, **caractérisé en ce que** le détecteur d'état (6) est relié au détecteur de fibrillation (1) et est agencé pour détecter la fibrillation ventriculaire comme indicateur ou comme l'un des indicateurs.

10. Dispositif selon la revendication 8 ou 9, **caractérisé en ce que** le détecteur d'état (6) est agencé pour détecter un débit cardiaque comme indicateur ou comme l'un des indicateurs.

11. Dispositif selon la revendication 10, **caractérisé en ce que** le détecteur d'état (6) est agencé pour détecter le débit cardiaque au moyen de mesures d'impédance épicardiaque ou endocardiaque.

12. Dispositif selon la revendication 8, 9, 10 ou 11, **caractérisé en ce que** le détecteur d'état (6) est agencé pour détecter une tension sanguine comme indicateur ou comme l'un des indicateurs.

13. Dispositif selon l'une des revendications précédentes, **caractérisé par** des moyens pour le déclenchement manuel d'une défibrillation auriculaire depuis l'extérieur du corps, qui sont reliés au moins indirectement au défibrillateur (2) et qui sont agencés pour faire déclencher une défibrillation par un patient même dans le cas où le détecteur de fibrillation (1) n'a pas encore détecté une fibrillation.

14. Dispositif selon la revendication 13, **caractérisé en ce que** le moyen de commande (4) comprend les moyens pour le déclenchement manuel d'une défibrillation auriculaire.
